# EUROPEAN PATENT SPECIFICATION

(11) **EP 2 470 224 B1**
(45) Date of publication and mention of the grant of the patent: **17.06.2015**
(21) Application number: 10749672.1
(22) Date of filing: 26.07.2010
(51) Int. Cl.: A61L 15/32

(54) **COLLAGENOUS MATERIAL**
KOLLAGENMATERIAL
MATÉRIAU COLLAGÈNE

(30) Priority: 28.08.2009 GB 0915049
(43) Date of publication of application: 04.07.2012
(73) Proprietor: Covidien AG, 8212 Neuhausen am Rheinfall (CH)
(72) Inventor: ARMITAGE, Paul, West Yorkshire WF10 2DB (GB); DAWSON, Christine, Elizabeth, West Yorkshire WF10 2DB (GB)
(74) Representative: Wilson Gunn
(86) International application number: PCT/GB2010/001410
(87) International publication number: WO 2011/023925

(56) References cited:
- WO-A1-00/29484
- WO-A1-85/05274
- WO-A1-95/32623
- US-A- 3 157 524

## Description

The present invention relates to a collagenous material and to a process for the manufacture thereof. The collagenous material may be used, for example, in wound care.

Collagen is a triple-helix protein which forms the major part of the dermal extracellular matrix (ECM), together with glycosaminoglycans, proteoglycans, laminin, fibronectin, elastin and cellular components. The ECM is the largest component of the dermal skin layer and the synthesis of ECM is a key feature of wound healing, especially where there has been a significant loss of tissue that precludes closure by primary intention.

The principal function of collagen in the dermal ECM is to act as a scaffold in connective tissue. Predominantly, collagen is present in the form of type I collagen (80-85%) and type III collagen (8-11%), both of which are fibrillar or rod-shaped collagens. The tensile strength of skin is due largely to these collagen molecules assembling into fibrils, with adjacent molecules crosslinking to further increase tensile strength. However, collagen laid down during wound healing has a reduced structural integrity compared to unwounded tissue; scar tissue rarely exceeds 70% of unwounded tissue strength.

In addition to being the main component of scar tissue, collagen has a key role in the control of the inflammatory response to injury and subsequent repair, with functions that influence cellular mitogenesis, differentiation and migration; protein synthesis in the ECM; synthesis and release of inflammatory cytokines and growth factors; and interactions between enzymes which remodel the ECM, including matrix metalloproteinases (MMPs) and their tissue inhibitors (TIMPs).

For medical applications, purified fibrous collagen has long been known to be a useful therapeutic adjunct to aid wound healing. Research into collagen-based dressing materials has shown that collagen significantly increases the production of fibroblasts in wounds, encouraging direct migration of cells into the wound enhancing the formation of new, organised collagen fibres.

Purified fibrous collagen pads have also been used to provide haemostatic dressings. When in contact with a bleeding surface, a fibrous collagen haemostat attracts platelets which adhere to collagen fibrils and release coagulation factors. This coagulation and aggregation of the platelets into thrombi on the collagenous mass, provides the formation of a physiologic platelet plug which slows and eventually stops the bleeding. The collagen fibrils also provide a structural matrix strengthening the platelet plug.

Previous studies have demonstrated that collagenous materials prepared using a process that retains the original fibre architecture and molecular ultrastructure of the natural tissues from which they are derived have a number of advantageous properties. For example, US 5397353 discloses a process for the preparation of collagenous materials that are substantially non-antigenic and substantially free of non-fibrous tissue proteins, cellular elements, lipids and lipid residues. The materials are typically sheet structures and are useful, for example, as implants. The collagenous sheet materials are susceptible to colonisation and vascularisation by host cells following implantation and are resistant to calcification.

It has further been shown that the favourable properties of these collagen sheet materials can be retained when the collagen material is presented in the form of particles comprising fibre fragments. EP 1112096 describes an injectable or mouldable composition of collagen fibre fragments prepared from the relatively large-scale sheet materials of US 5397353 in such a way as to retain the natural collagen fibre architecture and molecular ultrastructure. This size reduction is achieved by careful grinding or milling of the collagenous sheet materials, which may then be suspended to form a paste or injectable composition, as appropriate. WO00/29484 describes a high density collagen material, e.g. for reducing bleeding. The material is obtained by grinding of purified collagen into particles. Said particles are acidified by mixing them with a weak organic acid, thereby forming a paste. The paste is shaped by means of a mold and subsequently dried.

The present invention provides a novel collagenous material particularly suitable for use as a wound dressing.

According to a first aspect of the present invention there is provided a process for the manufacture of a collagenous material comprising the steps of:
(i) treating a natural tissue material with an organic solvent;
(ii) treating the natural tissue material with a proteolytic enzyme, so as to provide a treated material that is substantially free of non-fibrous tissue proteins, cellular elements and lipids or lipid residues,
(iii) forming a plurality of collagen particles from treated material said collagen particles comprising fragments of collagen fibres displaying original collagen fibre architecture and molecular ultrastracture of said natural tissue material;
(iv) treating the collagen particles with an aqueous acid solution to swell the collagen particles; and
(v) collectively dehydrating the swollen collagen particles.

By dehydrating the mass of swollen collagen particles, a coherent collagenous material is formed. The collagenous material has excellent properties for use as a wound dressing or as a component of a wound dressing. This porous, biocompatible material is non-antigenic and of natural origin, and provides a skin/tissue-like feel which improves patient acceptance and satisfaction. The collagenous material is soft and conformable, which is clearly advantageous for application to wound sites. Significantly, the collagenous material has good inherent strength and may be wrapped around wound areas to form an effective barrier. The collagenous material maintains a moist wound environment to promote wound healing, and its application helps to reduce contraction and scar tissue formation. Unlike a number of existing materials used in wound dressings, the collagenous material does not gel on wound contact.

Further, the presence of collagen at a wound site is known to increase the rate of wound healing.

Thus, the collagenous material described herein is particularly useful in wound care. The collagenous material may be presented in a hydrated state. The process may therefore further comprise at least one rehydration step, in which the collagenous material is maintained in an aqueous solution. For instance, the collagenous material may be maintained in saline, such as 0.9% saline.

The presentation of the collagenous material in a hydrated form helps to maintain a moist wound environment when the material is applied as a wound dressing. Surprisingly, it has been found that the collagenous material formed from collagen particles remains intact following processing/manufacture and does not return to a particulate state following rehydration.

The coherent collagenous material produced using the process of the present invention may be manufactured in any size, and can therefore be used to provide coverage of large wound sites, such as bums. The structural integrity of the collagenous material provides good protection to a wound site and the may be readily secured in position to prevent migration.

Although the processing typically results in some disruption to the structure of the collagen, the disruption is less than in prior art processes, which involve harsh chemical and mechanical treatments that significantly disrupt the collagen substructure to leave only collagen fragments in the form of tropocollagen (see, for example, US 3157524 and US 4412947). In contrast, collagenous materials manufactured according to the process of the present invention show good retention of collagen fibril structure and alignment, at least in part.

The natural tissue material may comprise any collagen-containing tissue material of human or animal origin. The natural tissue material may be a tissue comprising predominantly type I collagen. Preferred starting materials include dermis and tendons. In some embodiments, it is preferred that porcine tissue materials are processed to provide the collagenous material, although it will be understood that other mammalian sources may alternatively be employed, such as, for example, primates, cows, sheep, goats or horses.

Depending upon the starting material, the particles of collagen may contain a proportion of elastin. Thus, the particles, and the coherent collagenous materials formed therefrom, consist essentially of collagen optionally with small proportions of elastin.

The particles of acellular collagen may be formed using any suitable process. The original collagen fibre architecture and molecular ultrastructure of the natural tissue material must be retained in the particles. Preferred processes for preparing the collagen particles are therefore analogous to those described in EP 1112096. Preferably, collagenous material prepared in the form of a sheet or other large-scale structure is milled to form the particles. The collagenous material may be prepared by a process analogous to those described in US 5397353.

The collagenous tissue is neither solubilised nor denatured in the process, so its natural structure is maintained.

Thus, freshly cut natural tissue material may be treated to remove therefrom substantially all lipids and lipid residues and thereafter treated to remove non-fibrous tissue proteins and cellular elements.

The lipid extraction may be achieved by solvent extraction using an organic solvent, such as acetone. Other non-limiting examples of suitable solvents include non-aqueous solvents such as ethanol and ether.

Non-fibrous tissue proteins include glycoproteins, proteoglycans, globular proteins and the like. Cellular elements include antigenic proteins and enzymes and other cellular debris arising from the processing conditions. These portions of the natural tissue material may be removed by treatment with the proteolytic enzyme, such as trypsin. It has previously been found that above 20°C treatment with trypsin can in some circumstances result in an alteration of the collagen fibre structure leading to a lower physical strength. Moreover, low temperatures discourage the growth of microorganisms in the preparation. It is therefore preferred to carry out the treatment with trypsin at a temperature below 20°C. Moreover, trypsin is more stable below 20°C and lower amounts of it may be required. Any suitable trypsin concentration may be used, for instance a concentration within the range of around 0.01g/l to 25g/l. It has been found that good results can be obtained using about 2.5g/l trypsin.

Further treatments may optionally be carried out, such as treatment with one or more additional enzymes, for example a carbohydrate-splitting enzyme.

Those substances said to be "substantially free" of materials generally contain less than 5% of, and preferably less than 1%, of said materials.

The resulting collagenous material is then reduced to particles, care being taken to ensure that the size reduction is not associated with a degradation of the original collagen fibre architecture and molecular ultrastructure of the starting material. The particles may be produced by grinding or milling using, for example, a ball or hammer mill, which may be cooled to an appropriate temperature. The sheet material may be cut into small pieces prior to milling. Milling may be carried out in dry form (less than 10% moisture content) or in frozen hydrated form (20-80% moisture content).

The collagen particles may be of any suitable size. Typically, the collagen particles have a mean diameter within the range of from around 5µm to around 1000µm, more typically from around 50µm to around 500µm. Good results have been achieved using collagen particles with a mean diameter of approximately 150µm.

Any suitable aqueous acid solution may be used to treat the collagen particles. The choice of acid employed is not critical since the purpose is to lower the pH so that individual collagen particles readily absorb water. A weak organic or inorganic acid will generally be used. Non-limiting examples include dilute hydrochloric acid, acetic acid, and boric acid. Particularly good results have been observed using glacial acetic acid.

The concentration of the acid is typically at least 0.1% in order to provide a suitable pH for swelling. Acid can have a detrimental effect on the structure of collagen and therefore care should be taken to ensure that the aqueous acid solution is not too concentrated. The acid concentration should generally not exceed 1%. Typically, the pH is between 1 and 4, and more typically is between 2 and 4. Particularly good results have been achieved at around pH 3 to 3.5.

Typically, treatment of the collagen particles with the aqueous acid solution involves suspending the particles in the aqueous acid solution. The collagen particles may be weighed to determine the amount of aqueous acid solution required. Typically, solid collagen content may vary from about 0.5% to 5% of the aqueous acid solution volume. For example, good results have been observed using a formulation of 1% collagen in aqueous acid solution. The collagen particles are treated with the aqueous acid solution for sufficient time to allow swelling of the particles. This may take up to 24 hours or more.

In preferred embodiments, however, the collagen particles are suspended in the aqueous acid solution and mixed to accelerate swelling and to give an even distribution thereby ensuring relatively uniform end products.

Mixing can be achieved by various means, including mixing by hand or using mechanical mixing apparatus. For example, mixing can be carried out by homogenising, agitating, shaking, or blending. Preferably, the collagen particles are distributed in the aqueous acid solution by homogenisation.

Homogenisation of the suspension of swollen collagen particles results in a dispersion in the form of a slurry or 'paste' of swollen collagen particles. If fully dispersed, the combination of swollen collagen particles and aqueous acid solution may be considered a colloid.

Typically, solid collagen content may vary from about 0.5% to 5% of the solution volume. As a consequence of the reduced pH, the collagen particles swell in the aqueous acid solution and the increase in particle size reduces the fluidity of the dispersion. At concentrations of, for example, around 0.5 to 1% the dispersion can be easily mixed and poured. However, higher concentrations of collagen become difficult to mix due to the considerable increase in viscosity. At concentrations of about 2% and above, the dispersion may be relatively difficult to mix and generally cannot be poured.

In preferred embodiments, dehydration is carried out by treatment of the swollen collagen particles with a dehydrating agent. Any suitable dehydrating agent may be used. For example, the dehydrating agent may comprise any water miscible solvent that does not react with or dissolve collagen. Non-limiting examples of suitable dehydrating agents include non-aqueous solvents such as acetone, ethanol, ether, or mixtures thereof. The volume of solvent required to dehydrate the mass of swollen collagen particles may vary according to the solvent(s) used and the volume of collagen present. By way of example, acetone may be used at a ratio of about 5:1 solvent to collagen by volume. Multiple solvent rinses may be required to ensure complete removal of water. For instance, good results have been achieved using 3 x 45-minute rinses with acetone.

The solvent-dehydrated collagenous material is porous, with a sponge-like appearance.

In alternative embodiments, the swollen collagen particles may be dehydrated by air drying. For example, the mass of swollen collagen particles may be left at room temperature to allow air drying. Typically, air drying is carried out for at least about 24 hours, although for complete dehydration a period of about 48 hours or more may be required.

The collagenous material dehydrated by air-drying is porous, with a translucent, foam-like appearance which differs from the solvent-dehydrated collagenous material.

Other means of dehydrating may also be used, such as vacuum drying.

The process may optionally include a step of collectively shaping the swollen collagen particles prior to dehydration, the mass of swollen collagen particles then being dehydrated in the selected shape or conformation. It has been found that the shape or conformation of the dehydrated collagenous material is essentially determined by the shape or conformation of the collection of swollen collagen particles during the dehydration step. In other words, dehydration to some extent 'fixes' the shape or conformation of the final collagenous material. Usefully, therefore, the swollen collagen particles may be collectively shaped so that the resulting shape or conformation of the mass of swollen collagen particles is retained, or at least partly retained, in the collagenous material after dehydration.

Typically, the swollen collagen particles are presented in the form of a suspension, slurry or paste, in conjunction with the aqueous acid solution. The swollen collagen particles may, for example, be moulded, formed or cast into the desired shape prior to dehydration.

In a particularly preferred embodiment, the swollen collagen particles are formed into a sheet, film or similar shape. This may be achieved by casting the swollen collagen particles or by placing them into a suitable mould.

Other non-limiting examples of suitable shapes include beads, ropes or variations thereof.

It is desirable to maintain the shape or conformation of the collection of swollen collagen particles during dehydration. Although shape or conformation may be held in any suitable manner, freezing has been found to be a particularly convenient way to maintain the shape or conformation before and during the dehydration step. Therefore, after shaping, the collection of swollen collagen particles may optionally be frozen. Any means of freezing may be used. For instance, freezing may be carried out in an industrial, laboratory or domestic freezer, or by blast freezing or cryogenic freezing.

Optionally, cross-linking may be carried out to impart additional physical strength to the collagenous material, and an increased resistance to digestive enzymes that may be present in a wound healing environment.

The swollen collagen particles may be cross-linked before the dehydration step and/or concurrently therewith. Additionally or alternatively, the collagenous material may be cross-linked after dehydration.

Whilst any cross-linking agent may be used, preferred cross-linking agents include polyisocyanates, in particular diisocyanates. The polyfunctional isocyanates react with amino or hydroxyl groups of different protein chains so forming a material which has a stable structure retaining the original architecture of the collagen and which is resistant to enzymatic attack. It is known that antigenicity is associated with the amino groups of the protein chains of collagen, and reacting the amino groups with isocyanate removes any antigenicity associated with these groups. Preferred diisocyantes include aliphatic, aromatic and alicyclic diisocyanates as exemplified by 1,6-hexamethylene diisocyanate, toluene diisocyanate, 4,4'-diphenylmethane diisocyanate, and 4,4'-dicyclohexylmethane diisocyanate, respectively. A particularly preferred diisocyanate is hexamethylene diisocyanate (HMDI). Carbodiimide cross-linking agents may also be used, such as 1-ethyl-3-[3-dimethylaminopropyl]carbodiimide hydrochloride (EDC).

Where the cross-linking agent is used to treat non-dehydrated collagen material, a surfactant may be used to ensure proper dispersion of the cross-linking agent.

The extent of cross-linking may be varied. Usefully, this provides a mechanism for controlling the rate at which the collagenous material is resorbed or degraded during use. The resistance to degradation tends to increase as the extent of cross-linking is increased.

By way of example, cross-linking may be carried out using HMDI. As a guide, the HMDI may be used at a concentration of around 0.01g to 0.5g per 50g of collagen. If the concentration is too high, this may result in over-cross-linking and foreign body reactions. Cross-linking may be carried out for a range of different time periods. By way of example, the collagen may be exposed to the cross-linking agent for between around 1 hour and around 3 days. Typically, cross-linking is carried out for at least 12 hours, preferably at least 20 hours.

It will be appreciated that the cross-linking conditions may routinely be varied in order to adjust the extent of cross-linking.

According to a further aspect of the present invention there is provided a process for the manufacture of a collagenous sheet material from collagen particles, wherein said collagen particles are derived from a natural tissue material and are substantially free of non-fibrous tissue proteins, cellular elements and lipids or lipid residues, and wherein said collagen particles comprise fragments of collagen fibres displaying original collagen fibre architecture and molecular ultrastracture of said natural tissue material, said process comprising steps of:
suspending the collagen particles in an aqueous acid solution to swell the collagen particles;
collectively shaping the swollen collagen particles into a sheet; and
dehydrating the sheet of swollen collagen particles.

Optionally, the process may incorporate the additional steps as described herein.

According to a further aspect of the present invention there is provided a collagenous material obtainable by a process as herein described.

A range of different factors may be added to the collagenous material, such as growth factors, clotting agents, or other pharmaceutically active agents. The collagenous material may be seeded with cells, such as stem cells.

The collagenous material is useful in wound care. The collagenous material may also be used as a haemostat, to reduce or prevent blood flow from a body site.

According to a further aspect of the present invention there is provided a collagenous material as herein described for use in therapy.

According to a further aspect of the present invention there is provided the use in therapy of a collagenous material as herein described.

According to a further aspect of the present invention there is provided a collagenous material as herein described for use in wound care.

According to a further aspect of the present invention there is provided the use in wound care of a collagenous material as herein described.

According to a further aspect of the present invention there is provided a method of treatment of a wound comprising the step of applying to the wound a collagenous material as herein described.

According to a further aspect of the present invention there is provided a collagenous material as herein described for use as a haemostat.

According to a further aspect of the present invention there is provided the use in haemostasis of a collagenous material as herein described.

According to a further aspect of the present invention there is provided a method of reducing bleeding from a body site comprising the step of applying to the body site a collagenous material as herein described.

Embodiments of the present invention will now be described further in the following non-limiting examples and with reference to the accompanying drawing, in which:

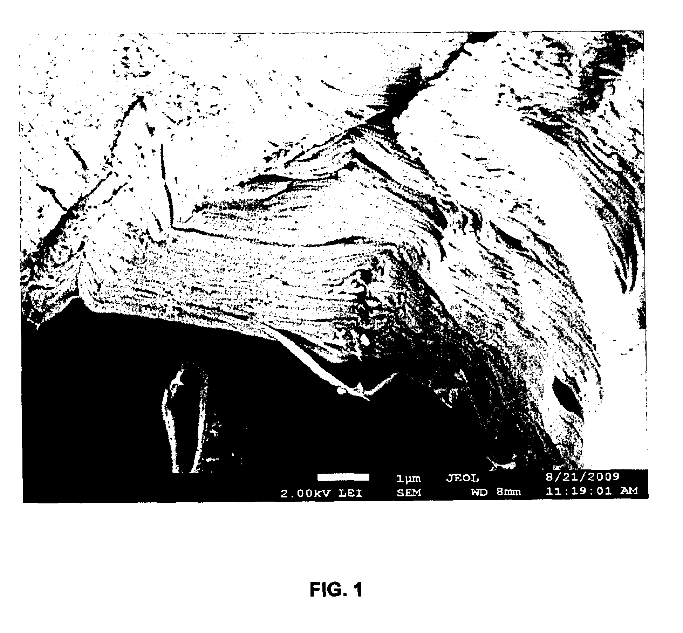

- Fig. 1: is a scanning electron micrograph at x10000 magnification of a representative sample of the collagenous material according to the present invention.

### Examples

### 1. Manufacture of solvent-dehydrated collagenous sheet material

Acellular collagen sheet material was prepared according to the method disclosed in US 5397353 and reduced to particles as described in EP 1112096.

Thus, freshly cut dermis harvested from sows was immersed in acetone. After 1 hour, the acetone was removed and replaced by fresh acetone. After a further incubation for around 36 hours, the tissue was removed from the acetone and placed in 0.9% saline to extract residual acetone. The tissue was then digested for 28 days with a solution of trypsin at a concentration of 2.5 mg/ml in 0.1 M phosphate buffer with 0.5 mg/ml sodium azide as a bacteriostatic agent. The purified tissue was removed from the trypsin solution and rinsed in buffer.

The resulting collagenous sheet material was cut into small pieces (approximately 5mm x 10mm in size) before being milled cryogenically to a mean particle size of around 130µm.

The collagen particles were suspended in a 0.6% glacial acetic acid solution such that a concentration of about 1% solid collagen was formulated. This suspension was homogenised for about 30 seconds using a Silverson^{®} L4RT homogeniser to evenly disperse the swollen collagen particles throughout the suspension, thereby forming a slurry or 'paste' of swollen collagen particles.

This dispersion was then cast into a pre-formed polystyrene mould and frozen at -20°C in a laboratory freezer. Once frozen, the material was removed from the mould and defrosted in acetone to give a dehydrated collagenous sheet. Several acetone rinses were performed to ensure complete dehydration of the collagen.

The collagenous sheet material was then cross-linked using HMDI in acetone. A concentration of about 0.1g HMDI per 50g of solid collagen was added. The material was cross-linked for at least 20 hours, rinsed in acetone, and allowed to air dry.

The collagenous material is porous, with a sponge-like appearance.

Upon rehydration, the collagenous sheet material did not gel or fragment, and had good structural integrity.

### 2. Manufacture of air-dried collagenous sheet material

The process of Example 1 was followed up to the step of casting the collagen dispersion into the pre-formed polystyrene mould.

There followed a step of air-drying at room temperature for a period of about 48 hours.

The resulting material had a porous, translucent, foam-like appearance which differed from the collagenous material of Example 1.

Upon rehydration, the collagenous sheet material did not gel or fragment, and had good structural integrity.

### 3. SEM analysis

The collagenous material of Example 1 was examined by SEM. Samples of approximately 7mm x 7mm were cut from the material and mounted on SEM stubs using double-sided sticky tabs. Silver-dag was used around the edges to reduce charge effects. The samples were sputter coated with approximately 5nm of gold/palladium. They were viewed at 2kV using a JEOL JSM-7500F scanning electron microscope at a working distance of approximately 8mm and the LEI detector.

Results are shown in the scanning electron micrograph of Fig. 1. It can be seen that, at least in part, the collagenous material showed good retention of collagen structure and alignment seen in the particulate collagen starting material.

### 4. Tensile strength testing

The tensile strength of the collagenous materials of Examples 1 and 2 was tested using a Hounsfield tensiometer equipped with a 1kN load cell. Jaw speed was set at 10mm/min, with samples tested until failure. For reference, testing was repeated using samples of Promogran^{®}, an existing wound care product comprising a freeze-dried composite of oxidised regenerated cellulose and collagen.

Testing was carried out using representative 100mm x 10mm samples, fully hydrated in 0.9% saline. Maximum load before failure was recorded to provide ultimate tensile strength in Newtons.

The collagenous material of Example 1 was found to have a tensile strength of around 0.3N in the hydrated state and the collagenous material of Example 2 had a tensile strength of around 0.9N in the hydrated state. The hydrated Promogran^{®} material had a tensile strength of around 0.1 N.

It is of course to be understood that the invention is not intended to be restricted by the details of the above specific embodiments, which are provided by way of example only.

## Claims

1. A process for the manufacture of a collagenous material, comprising the steps of:
(i) treating a natural tissue material with an organic solvent;
(ii) treating the natural tissue material with a proteolytic enzyme,
so as to provide a treated material that is substantially free of non-fibrous tissue proteins, cellular elements and lipids or lipid residues;
(iii) forming a plurality of collagen particles from the treated material, said collagen particles comprising fragments of collagen fibres displaying the original collagen fibre architecture and molecular ultrastracture of said natural tissue material;
(iv) treating the collagen particles with an aqueous acid solution to swell the collagen particles; and
(v) collectively dehydrating the swollen collagen particles.

2. A process according to claim 1, wherein the proteolytic enzyme comprises trypsin.

3. A process according to claim 1 or claim 2, wherein the collagen particles are suspended in the aqueous acid solution.

4. A process according to any one of claims 1 to 3, comprising a step of mixing the collagen particles in the aqueous acid solution.

5. A process according to claim 4, wherein the mixing step comprises homogenising the collagen particles in the aqueous acid solution.

6. A process according to any one of the preceding claims, wherein the step of collectively dehydrating the swollen collagen particles comprises treating the swollen collagen particles with a dehydrating agent.

7. A process according to claim 6, wherein the dehydrating agent comprises a water miscible solvent.

8. A process according to any one of claims 1 to 5, wherein the step of collectively dehydrating the swollen collagen particles comprises air drying.

9. A process according to any one of the preceding claims, wherein the process comprises a step of collectively shaping the swollen collagen particles prior to dehydration.

10. A process according to claim 9, wherein the swollen collagen particles are collectively shaped into a sheet.

11. A process according to claim 9 or claim 10, wherein the process comprises a step of freezing the collectively shaped swollen collagen particles.

12. A process according to any one of the preceding claims, wherein the process comprises a step of cross-linking the swollen collagen particles prior to and/or concurrently with dehydration.

13. A process according to any one of the preceding claims, wherein the process comprises a step of cross-linking the collagenous material after dehydration.

14. A process according to any one of the preceding claims, wherein the process comprises a step of rehydrating the collagenous material.

15. A collagenous material obtainable by a process according to any one of the preceding claims.

16. A collagenous material according to claim 15 for use in therapy.

## Patentansprüche

1. Verfahren zum Herstellen eines kollagenen Materials, bei welchem
(i) ein natürliches Gewebematerial mit einem organischen Lösungsmittel behandelt wird,
(ii) das natürliche Gewebematerial mit einem proteolytischen Enzym behandelt wird, um ein behandeltes Material zur Verfügung zu stellen, welches im Wesentlichen frei von nicht-fibrösen Gewebeproteinen, zellulären Bestandteilen und Lipiden oder Lipidrückständen ist,
(iii) eine Mehrzahl von Kollagenpartikeln aus dem behandelten Material gebildet wird, wobei die Kollagenpartikel Fragmente von Kollagenfasern aufweisen, welche die ursprüngliche Kollagenfaserarchitektur und molekulare Ultrastruktur des natürlichen Gewebematerials zeigen,
(iv) die Kollagenpartikel mit einer wässrigen Säurelösung behandelt werden, um die Kollagenpartikel aufzuquellen, und
(v) die aufgequollenen Kollagenpartikel gemeinsam dehydriert werden.

2. Verfahren nach Anspruch 1, wobei das proteolytische Enzym Trypsin aufweist.

3. Verfahren nach Anspruch 1 oder 2, wobei die Kollagenpartikel in der wässrigen Säurelösung suspendiert sind.

4. Verfahren nach einem der Ansprüche 1 bis 3, welches einen Schritt des Beimischens der Kollagenpartikel in die wässrige Säurelösung aufweist.

5. Verfahren nach Anspruch 4, wobei der Beimischungsschritt das Homogenisieren der Kollagenpartikel in der wässrigen Säurelösung aufweist.

6. Verfahren nach einem der vorangehenden Ansprüche, wobei der Schritt des gemeinsamen Dehydrierens der gequollenen Kollagenpartikel die Behandlung der gequollene Kollagenpartikel mit einem dehydrierenden Reagens umfasst.

7. Verfahren nach Anspruch 6, wobei das dehydrierende Reagens ein Lösungsmittel aufweist, welches mit Wasser mischbar ist.

8. Verfahren nach einem der Ansprüche 1 bis 5, wobei der Schritt des gemeinsamen Dehydrierens der gequollenen Kollagenpartikel Lufttrocknen aufweist.

9. Verfahren nach einem der vorangehenden Ansprüche, wobei das Verfahren einen Schritt des gemeinsamen Formens der gequollenen Kollagenpartikel vor der Dehydratation aufweist.

10. Verfahren nach Anspruch 9, wobei die gequollenen Kollagenpartikel gemeinsam zu einem Blatt geformt werden.

11. Verfahren nach Anspruch 9 oder 10, wobei das Verfahren den Schritt des Einfrierens der gemeinsam geformten, gequollenen Kollagenpartikel aufweist.

12. Verfahren nach einem der voranstehenden Ansprüche, wobei das Verfahren den Schritt des Cross-Linkens der gequollenen Kollagenpartikel vor und/oder während der Dehydratation aufweist.

13. Verfahren nach einem der voranstehenden Ansprüche, wobei das Verfahren den Schritt des Cross-Linkens des kollagenen Materials nach der Dehydratation aufweist.

14. Verfahren nach einem der voranstehenden Ansprüche, wobei das Verfahren den Schritt der Rehydratation des kollagenen Materials aufweist.

15. Ein kollagenes Material, welches durch ein Verfahren nach einem der voranstehenden Ansprüche erhältlich ist.

16. Ein kollagenes Material nach Anspruch 15, zur Verwendung in therapeutischen Verfahren.

## Revendications

1. Procédé pour la fabrication d'un matériau collagène, comprenant les étapes suivantes :
(i) Traiter un tissu naturel avec un solvant organique ;
(ii) Traiter le tissu naturel par une enzyme protéolytique, de manière à obtenir un matériau traité qui est substantiellement dépourvu de protéines tissulaires non fibreuses, d'éléments de cellules, de lipides ou de résidus de lipides ;
(iii) Former une pluralité de particules de collagène à partir du matériau traité, lesdites particules de collagène incluant des fragments de fibres de collagène présentant l'architecture initiale de la fibre collagène et l'ultrastructure moléculaire du tissu naturel;
(iv) Traiter les particules de collagène avec une solution aqueuse acide pour faire gonfler les particules de collagène ; et
(v) Déshydrater collectivement les particules de collagène gonflées.

2. Procédé selon la revendication 1, dans lequel l'enzyme protéolytique comporte de la trypsine.

3. Procédé selon la revendication 1 ou 2, dans lequel les particules de collagène sont en suspension dans la solution acide aqueuse.

4. Procédé selon l'une quelconque des revendications 1 à 3, incluant une étape de mélange des particules de collagène dans la solution acide aqueuse.

5. Procédé selon la revendication 4, dans lequel l'étape de mélange intègre l'homogénéisation des particules de collagène dans la solution acide aqueuse.

6. Procédé selon l'une quelconque des revendications précédentes, dans lequel l'étape de déshydratation collective des particules de collagène gonflées inclut le traitement desdites particules de collagène gonflées avec un agent déshydratant.

7. Procédé selon la revendication 6, dans lequel l'agent déshydratant inclut un solvant miscible à l'eau.

8. Procédé selon l'une quelconque des revendications 1 à 5, dans lequel l'étape de déshydratation collective des particules de collagène gonflées comporte un séchage à l'air.

9. Procédé selon l'une quelconque des revendications précédentes, incluant une étape consistant à mettre en forme collectivement les particules de collagène gonflées, avant leur déshydratation.

10. Procédé selon la revendication 9, dans lequel les particules de collagène gonflées sont collectivement mises en forme pour obtenir une feuille.

11. Procédé selon les revendications 9 ou 10, incluant une étape consistant à geler les particules de collagène gonflées et collectivement mises en forme.

12. Procédé selon l'une quelconque des revendications précédentes, incluant une étape consistant à lier entre elles les particules de collagène gonflées, avant et/ou en même temps que la déshydratation.

13. Procédé selon l'une quelconque des revendications précédentes, incluant une étape consistant à lier entre elles les particules de collagène gonflées, après la déshydratation.

14. Procédé selon l'une quelconque des revendications précédentes, incluant une étape de réhydratation du matériau collagène.

15. Matériau collagène qui peut être obtenu par un procédé selon l'une quelconque des revendications précédentes.

16. Matériau collagène selon la revendication 15, pour une utilisation thérapeutique.
